# EUROPEAN PATENT APPLICATION

(11) **EP 1 072 687 A2**
(43) Date of publication of application: **31.01.2001**
(21) Application number: 00115161.2
(22) Date of filing: 12.07.2000
(51) Int. Cl.: C12Q 1/68, G01N 33/50, A61P 35/04

(54) **Method for testing metastatic potential and method for screening compounds with metastasis-suppressing activity**

(30) Priority: 12.07.1999 JP 19803299
(71) Applicant: Medical & Biological Laboratories Co., Ltd., Nagayo-shi, Aichi 460-0002 (JP)
(72) Inventor: Nojima, Hiroshi, Toyonaka-shi, Osaka 560-0005 (JP); Itoh, Akihiko, Toyonaka-shi, Osaka 565-0085 (JP)
(74) Representative: VOSSIUS & PARTNER

(57) **Abstract**

A method for testing the metastatic potential of cancer cells using the expression level of connexin 26 as the marker is provided. In addition, a method for screening metastasis-suppressing drugs based on the binding activity to connexin 26 is provided. The expression level of connexin 26 is a novel marker that elevates corresponding to the magnitude of the metastatic potential.

SEQUENCE LISTING

<110> Medical & Biological Laboratories co., Ltd.

<120> Method for testing metastatic potential and method for screening
compounds with metastasis-suppressing activity

<130> M3-105

<140>
<141>

<150> JP 1999-198032
<151> 1999-07-12

<160> 4

<170> PatentIn Ver. 2.0

<210> 1
<211> 2311
<212> DNA
<213> Homo sapiens

<220>
<221> CDS
<222> (199)..(876)

<400> 1
gatttaatcc tatgacaaac taagttggtt ctgtcttcac ctgttttggt gaggttgtgt 60

aagagttggt gtttgctcag gaagagattt aagcatgctt gcttacccag actcagagaa 120

gtctccctgt tctgtcctag ctatgttcct gtgttgtgtg cattcgtctt ttccagagca 180

aaccgcccag agtagaag atg gat tgg ggc acg ctg cag acg atc ctg ggg 231
Met Asp Trp Gly Thr Leu Gln Thr Ile Leu Gly
1               5                   10

ggt gtg aac aaa cac tcc acc agc att gga aag atc tgg ctc acc gtc 279
Gly Val Asn Lys His Ser Thr Ser Ile Gly Lys Ile Trp Leu Thr Val
            15                  20                  25

ctc ttc att ttt cgc att atg atc ctc gtt gtg gct gca aag gag gtg 327
Leu Phe Ile Phe Arg Ile Met Ile Leu Val Val Ala Ala Lys Glu Val
        30                  35                  40

tgg gga gat gag cag gcc gac ttt gtc tgc aac acc ctg cag cca ggc 375
Trp Gly Asp Glu Gln Ala Asp Phe Val Cys Asn Thr Leu Gln Pro Gly
    45                  50                  55

tgc aag aac gtg tgc tac gat cac tac ttc ccc atc tcc cac atc cgg 423
Cys Lys Asn Val Cys Tyr Asp His Tyr Phe Pro Ile Ser His Ile Arg
60                  65                  70                  75

cta tgg gcc ctg cag ctg atc ttc gtg tcc agc cca gcg ctc cta gtg 471
Leu Trp Ala Leu Gln Leu Ile Phe Val Ser Ser Pro Ala Leu Leu Val
                80                  85                  90

gcc atg cac gtg gcc tac cgg aga cat gag aag aag agg aag ttc atc 519

Ala Met His Val Ala Tyr Arg Arg His Glu Lys Lys Arg Lys Phe Ile
            95                  100                 105

aag ggg gag ata aag agt gaa ttt aag gac atc gag gag atc aaa acc 567
Lys Gly Glu Ile Lys Ser Glu Phe Lys Asp Ile Glu Glu Ile Lys Thr
        110                 115                 120

cag aag gtc cgc atc gaa ggc tcc ctg tgg tgg acc tac aca agc agc 615
Gln Lys Val Arg Ile Glu Gly Ser Leu Trp Trp Thr Tyr Thr Ser Ser
    125                 130                 135

atc ttc ttc cgg gtc atc ttc gaa gcc gcc ttc atg tac gtc ttc tat 663
Ile Phe Phe Arg Val Ile Phe Glu Ala Ala Phe Met Tyr Val Phe Tyr
140                 145                 150                 155

gtc atg tac gac ggc ttc tcc atg cag cgg ctg gtg aag tgc aac gcc 711
Val Met Tyr Asp Gly Phe Ser Met Gln Arg Leu Val Lys Cys Asn Ala
                160                 165                 170

tgg cct tgt ccc aac act gtg gac tgc ttt gtg tcc cgg ccc acg gag 759
Trp Pro Cys Pro Asn Thr Val Asp Cys Phe Val Ser Arg Pro Thr Glu
            175                 180                 185

aag act gtc ttc aca gtg ttc atg att gca gtg tct gga att tgc atc 807
Lys Thr Val Phe Thr Val Phe Met Ile Ala Val Ser Gly Ile Cys Ile
        190                 195                 200

ctg ctg aat gtc act gaa ttg tgt tat ttg cta att aga tat tgt tct 855
Leu Leu Asn Val Thr Glu Leu Cys Tyr Leu Leu Ile Arg Tyr Cys Ser
    205                 210                 215

ggg aag tca aaa aag cca gtt taacgcattg cccagttgtt agattaagaa 906
Gly Lys Ser Lys Lys Pro Val
220                 225

atagacagca tgagagggat gaggcaaccc gtgctcagct gtcaaggctc agtcgccagc 966

atttcccaac acaaagattc tgaccttaaa tgcaaccatt tgaaacccct gtaggcctca 1026

ggtgaaactc cagatgccac aatgagctct gctcccctaa agcctcaaaa caaaggccta 1086

attctatgcc tgtcttaatt ttctttcact taagttagtt ccactgagac cccaggctgt 1146

taggggttat tggtgtaagg tactttcata ttttaaacag aggatatcgg catttgtttc 1206

tttctctgag gacaagagaa aaaagccagg ttccacagag gacacagaga aggtttgggt 1266

gtcctcctgg ggttcttttt gccaactttc cccacgttaa aggtgaacat tggttctttc 1326

atttgctttg gaagttttaa tctctaacag tggacaaagt taccagtgcc ttaaactctg 1386

ttacactttt tggaagtgaa aactttgtag tatgataggt tattttgatg taaagatgtt 1446

ctggatacca ttatatgttc cccctgtttc agaggctcag attgtaatat gtaaatggta 1506

tgtcattcgc tactatgatt taatttgaaa tatggtcttt tggttatgaa tactttgcag 1566

cacagctgag agaggctgtc tgttgtattc attgtggtca tagcacctaa caacattgta 1626

gcctcaatcg agtgagacag actagaagtt cctagttggc ttatgatagc aaatggcctc 1686

atgtcaaata ttagatgtaa ttttgtgtaa gaaatacaga ctggatgtac caccaactac 1746

tacctgtaat gacaggcctg tccaacacat ctcccttttc catgctgtgg tagccagcat 1806

cggaaagaac gctgatttaa agaggtgagc ttgggaattt tattgacaca gtaccattta 1866

atggggagac aaaaatgggg gccaggggag ggagaagttt ctgtcgttaa aaacgagttt 1926

ggaaagactg gactctaaat tctgttgatt aaagatgagc tttgtctacc ttcaaaagtt 1986

tgtttggctt acccccttca gcctccaatt ttttaagtga aaatataact aataacatgt 2046

gaaaagaata gaagctaagg tttagataaa tattgagcag atctatagga agattgaacc 2106

tgaatattgc cattatgctt gacatggttt ccaaaaaatg gtactccaca tacttcagtg 2166

agggtaagta ttttcctgtt gtcaagaata gcattgtaaa agcattttgt aataataaag 2226

aatagcttta atgatatgct tgtaactaaa ataattttgt aatgtatcaa atacatttaa 2286

aacattaaaa tataatctct ataat 2311

<210> 2
<211> 226
<212> PRT
<213> Homo sapiens

<400> 2
Met Asp Trp Gly Thr Leu Gln Thr Ile Leu Gly Gly Val Asn Lys His
1               5                   10                  15

Ser Thr Ser Ile Gly Lys Ile Trp Leu Thr Val Leu Phe Ile Phe Arg
            20                  25                  30

Ile Met Ile Leu Val Val Ala Ala Lys Glu Val Trp Gly Asp Glu Gln
        35                  40                  45

Ala Asp Phe Val Cys Asn Thr Leu Gln Pro Gly Cys Lys Asn Val Cys
    50                  55                  60

Tyr Asp His Tyr Phe Pro Ile Ser His Ile Arg Leu Trp Ala Leu Gln
65                  70                  75                  80

Leu Ile Phe Val Ser Ser Pro Ala Leu Leu Val Ala Met His Val Ala
                85                  90                  95

Tyr Arg Arg His Glu Lys Lys Arg Lys Phe Ile Lys Gly Glu Ile Lys
            100                 105                 110

Ser Glu Phe Lys Asp Ile Glu Glu Ile Lys Thr Gln Lys Val Arg Ile
        115                 120                 125

Glu Gly Ser Leu Trp Trp Thr Tyr Thr Ser Ser Ile Phe Phe Arg Val
    130                 135                 140

Ile Phe Glu Ala Ala Phe Met Tyr Val Phe Tyr Val Met Tyr Asp Gly
145                 150                 155                 160

Phe Ser Met Gln Arg Leu Val Lys Cys Asn Ala Trp Pro Cys Pro Asn
                165                 170                 175

Thr Val Asp Cys Phe Val Ser Arg Pro Thr Glu Lys Thr Val Phe Thr
            180                 185                 190

Val Phe Met Ile Ala Val Ser Gly Ile Cys Ile Leu Leu Asn Val Thr
        195                 200                 205

Glu Leu Cys Tyr Leu Leu Ile Arg Tyr Cys Ser Gly Lys Ser Lys Lys
    210                 215                 220

Pro Val
225


<210> 3
<211> 30
<212> DNA
<213> Artificial Sequence

<220>
<223> Description of Artificial Sequence:Artificially
      Synthesized Primer Sequence

<400> 3
gcgaattcag gtgtaaattt accaaaaata                                    30


<210> 4
<211> 30
<212> DNA
<213> Artificial Sequence

<220>
<223> Description of Artificial Sequence:Artificially
      Synthesized Primer Sequence

<400> 4
gcgctcgagc acccaagctt tccatcctgg                                    30

## Description

### FIELD OF THE INVENTION

The present invention relates to a method for testing the metastatic potential and a method for screening compounds with metastasis suppressing activity.

### BACKGROUND OF THE INVENTION

Metastasis often occurs with the progression of cancer. Metastasis refers to a phenomenon in which cancer cells, which have proliferated in a tissue, detach themselves from primary focus, invade other sites and resume proliferation at those sites. When metastasized cancer cannot be cured, it will eventually lead to the death of patient. Metastatic potential acquired by cancer cells is generally referred to as the "malignancy" of these cells. Therefore, along with diagnostic methods that enable the early detection of cancer and treatment methods for resection of cancerous tissues, comprehension of the malignancy grade of the cancer and suppression of metastasis are also important research issues aimed at the conquest of cancer.

Cancer metastasis requires a series of sequential steps including the detachment of cancer cells from the primary focus, migration among tissues, and re-proliferation at the migrated site. In reality, complex mechanisms are thought to be involved in this series of processes, the general feature of which is yet to be fully understood. For example, it has been proposed that the activation of a group of proteases called matrix metalloproteases (MMP) are closely related to oncogenesis and malignancy of cancer. When cancer cells detach from their primary focus, permeate into blood vessels, and migrate to metastatic sites, the activity to disintegrate the extracellular matrix (ECM) is an important element. MMP has been indicated to be profoundly involved in disintegrating this ECM. In this respect, some MMP inhibitors have been being developed as candidates for anticancer agents. In addition, it has been reported that integrins, which are cell adhesion molecules, are involved in the metastatic activity and organ specificity at the site of metastasis.

However, as mentioned above, since much remain unsolved regarding metastasis, it is impossible to comprehend the general feature of metastatic processes based only on mechanisms so far revealed. For the development of antimetastatic drugs, it is necessary to elucidate metastatic mechanisms in more detail. Assuming that metastasis results from accumulation of several different steps, elucidation of factors required for achieving each step followed by comprehension of their organized activities may lead to the accurate assessment of metastatic potential. Thus, reliable protection from metastasis can be achieved by individually inhibiting major factors involved in each step. In particular, elucidation of factors supporting metastatic potential of cancers that are generally thought to be at high risk of developing metastasis, such as melanoma and lung small cell carcinoma, can contribute a great deal to their diagnosis and treatment.

Almost all the factors involved in metastasis so far reported have been found by the differential display method. There is a report of an attempt to isolate genes related to metastasis by the differential display method utilizing F10 and BL6 cells employed in examples of this invention described later (Cancer Res. 56, 875-879, 1996). However, most of the genes thus isolated are housekeeping genes, indicating low efficiency, a drawback of the differential display method.

The gap junction, which is an intercellular binding form observed in many animal tissues, is known as one type of cell-cell adhesion. In gap junctions, neighboring cells adhere to each other with gaps, forming an intercellular communication passage composed of connexon at the binding site. Low molecular weight substances of 1000 Da or less such as cAMP are allowed to migrate between cells via this communication passage, whereas ordinary proteins and nucleic acids are not. Using this phenomenon, an analytical method termed "gap junction assay" has been developed, in which one cell labeled with a fluorescent dye of the molecular weight of about 1000 to 1500 is brought into contact with another cell. If the gap junction is formed between the two, the fluorescent dye migration into the other cell (referred to as dye-coupling) is observed. Of gap junction assays, the one using a fluorescent dye as the marker may be called in particular the "dye-transfer assay".

Connexon is composed of connexins, a group of proteins which are structurally similar to each other. Twelve varieties of connexins have been so far identified, and are classified into α and β types. While it is known that a specific cell expresses its own particular connexin, connexins themselves are expressed in many different types of cells, and are evolutionarily well conserved. Therefore, connexins are considered to have important functions in the establishment of cell communities. Owing to the recent progress in molecular biology, relationship between connexins and some hereditary diseases has been revealed. For example, β1connexin/connexin 32 (Cx32) is a responsible gene of X-linked Charcot-Marie-Tooth disease (CMTX), a hereditary neurodegerative disease (Science 1993 Dec 24; 262 (5142): 2039-2042). In addition, α1connexin/connexin 43 (Cx43) is assumed to be a responsible gene of visceroatrial heterotaxia syndrome (VAH), a hereditary disease with symptoms including cardiac heterotaxia (N. Eng. J. Med. 1995 May 18; 332 (20): 1323-1329). Furthermore, it has been proved that β2 connexin/connexin 26 (Cx26) is a responsible gene of autosomal recessive non-syndromic deafness (DFNB1) (Nature 1997 May 1; 387 (6628): 80-83) and autosomal dominant non-syndromic deafness (DFNA) (Nature 1998 May 28; 393 (6683): 319-320), both of which are hereditary deafnesses.

It has been hypothesized that the signal for suppressing cell proliferation is directly transmitted to the neighboring cell through the gap junction. As if it were to support this hypothesis, connexin 26 (hereafter may be abbreviated as Cx26) was isolated as a gene expressed in normal mammary gland cells but not in breast cancer cells using the subtraction method (Proc Natl Acad Sci USA 1991 Apr 1; 88 (7): 2825-2829). In fact, proliferation of a breast cancer cell line was reportedly suppressed experimentaly by an overexpression of Cx26 *in vitro*, and its oncogenic activity was also significantly inhibited in nude mice, indicating that Cx26 functions as a tumor-supperesing gene in breast cancer. Cx26 also reportedly suppresses the proliferation of HeLa cells, and, at the same time, augments a bystander effect so that it kills not only the cells transduced with the gene for gene therapy, but also surrounding tumor cells (Cancer Res 1997 Jul 15; 57 (14): 2929-2932). However, both Cx26 and Cx43 are expressed in a primary brain carcinoma (Neurosurgery 1999 Feb; 44 (2): 361-369), while Cx26 is expressed very little in normal mammary glands, its expression is elevated in 56% of atypical breast cancers (J Pathol 1998 Jan; 184 (1): 37-43). Thus, at present, results obtained in artificial experiments do not necessarily coincide with those acquired using clinical samples.

### SUMMARY OF THE INVENTION

An objective of the present invention is to provide a novel marker reflecting metastatic potential of cancer cells. Another objective of this invention is to provide a method for testing the metastatic potential using said marker as well as a method for screening drugs that suppress metastasis.

To identify metastatic potential-determining factors, the present inventors performed subtraction of cDNA libraries between BL6 cells (naturally high-metastatic) and F10 cells (naturally low-metastatic) both derived from mouse and isolated twenty odd different genes highly expressed in BL6 cells. The subtraction method used was a strict method enabling the comprehensive and efficient cloning of a group of transcription-targeting genes. This method was developed by the present inventors (Kobori, M., Ikeda, Y., Nara, H., Kumegawa, M., Nojima, H. and Kawashima, H.: Large scale preparation of a subtracted cDNA library. Genes Cells 3, 459-475, 1998) and performs repeated subtractive differentiation of high quality cDNA libraries against the same driver mRNA. By this method, genes involved in metastasis can be concentrated to markedly improve the isolation efficiency.

Sublines of mouse melanoma B16 cells include BL6 cells, which have both experimental (via intravenous injection) and spontaneous (via subcutaneous injection) metastatic potentials, and F10 cells having the experimental metastatic potential but not the spontaneous one. Since the spontaneous metastatic potential is thought to be more closely related in properties to human malignant cancers, as compared with the experimental metastatic potential, these two types of potentials are a favorable combination for subtraction of this invention. The present inventors also found the presence of a definite relationship between some of the isolated genes, including connexin 26, and metastatic properties.

Furthermore, as a result of examining the spontaneous metastatic potential of F10 cells transduced with cloned full-length cDNAs of the above genes, a high metastasis equivalent to that of BL6 cells was observed only in F10 cells transduced with connexin 26. In addition, to confirm whether the above-described effect is actually due to the overexpression of connexin 26, a dominant negative form of connexin 26 was transferred into BL6 cells to test whether it suppresses the spontaneous metastatic potential intrinsic to BL6 cells. As a result, it was found that the spontaneous metastatic potential was progressively inhibited as the expression amount of said dominant negative form exceeded the amount of endogenous connexin 26 originally present in BL6 cells.

Based on this knowledge, the present inventors completed the present invention. Namely, this invention relates to methods for testing metastatic potential and screening metastasis-suppressing drugs, more specifically to:
(1) a method for testing metastatic potential, the method comprising the comparison of the expression level of the connexin 26 gene in cancer cells with that in normal cells;
(2) the testing method according to (1), wherein the expression level of the connexin 26 gene is assessed by quantifying the amount of mRNA encoding connexin 26;
(3) the testing method according to (2), wherein the region in said mRNA encoding at least the domain expressing the gap junction activity of connexin 26 is quantified;
(4) the testing method according to (1), wherein the expression level of connexin 26 gene in cancer cells is measured by quantifying connexin 26 protein contained in cancer cells;
(5) the testing method according to (4), wherein the expression level of connexin 26 gene is measured by an immunoassay using an antibody specifically recognizing the connexin 26 protein;
(6) a method for testing metastatic potential, the method comprising the assessment of the expression level of the connexin 26 gene based on the amount of the humoral connexin 26 protein;
(7) a reagent for testing the metastatic potential comprising a DNA that specifically hybridizes to the DNA comprising the nucleotide sequence shown in SEQ ID NO: 1, and comprising at least 15 nucleotides;
(8) a reagent for testing the metastatic potential comprising an antibody that specifically binds to a protein comprising the amino acid sequence shown in SEQ ID NO: 2;
(9) a method for screening metastasis suppressing agents comprising the steps of:
   a) providing a protein comprising a domain that exhibits at least the gap-junction activity of connexin 26;
   b) contacting said protein of a) with candidate compounds; and
   c) selecting candidate compounds that bind to the protein of a);
(10) the method of screening according to (9), wherein said candidate compounds are RNA libraries comprising random nucleotide sequences, and;
(11) a metastasis-suppressing drug comprising a compound isolable by the method according to (9) or (10) as an active ingredient.

Any patents, patent applications, and publications cited herein are incorporated by reference.

### BRIEF DESCRIPTION OF THE INVENTION

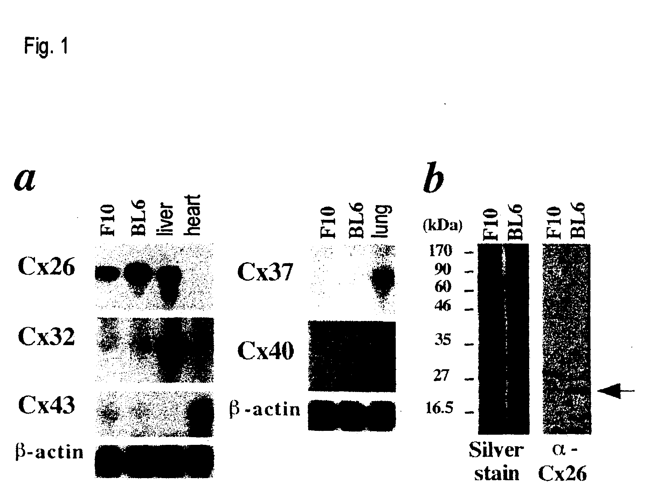

Fig. 1 shows the expression level of connexin 26 in F10 and BL6 cells detected by Nothern blot analysis.
   (a) Northern blot analysis to detect the transcripts of Cx26, Cx32, Cx43, Cx37, and Cx40. As a positive control, total RNA extracted from mouse liver, heart, and lung tissues were also blotted.
   (b) Expression of Cx26 protein in F10 and BL6 cells. Anti-Cx26 antibody detected monomeric Cx26 protein at approximately 24 kDa (indicated by an arrow in right panel). Equal protein loading was confirmed by silver staining of the gel (left panel).

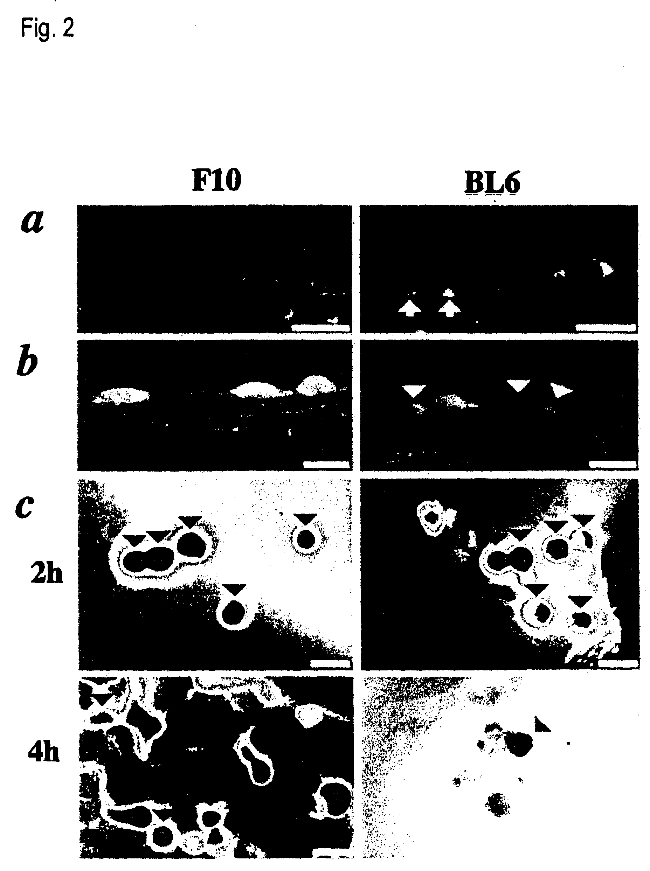

Fig. 2 shows micrographs of the co-culture system composed of an inferior vena cava (IVC) fragment and melanoma cells seeded onto it. Bar = 20 µm.
   (a) After 4 h, the co-culture with F10 (left panel) or BL6 (right panel) cells was immunoreacted with anti-Cx26 antibody and observed with a fluorescence microscope. Arrows indicate the localization of Cx26 near the cell membrane.
   (b) BCECF-labeled F10 (left panel) or BL6 (right panel) cells were seeded onto an IVC fragment. The co-culture was sectioned after 4 h and observed with a fluorescence microscope. BCECF-leakage is indicated by arrowheads.
   (c) The dye-spread was observed from directly above the co-culture at 2 (upper panel) and 4 (lower panel) h by confocal laser scanning microscope. Intensity of fluorescence is displayed by color; red indicates a greater intensity than blue. Arrowheads indicate BCECF-labeled F10 and BL6 cells.
Fig. 3 is a graph showing the results of the gap junction assay. The number of cells showing dye-coupling with endothelial cells was counted when five hundred BCECF-labeled cells were observed at 4 h. Data are expressed as the mean of three independent experiments. Bars are standard errors. *p< 0.01 by *t*-test when compared to the values obtained from F10 cells.
Fig. 4 shows micrographs of co-culture with BCECF-labeled cells at 6 h. Sections were observed directly with a fluorescence microscope (left panel) and with a light microscope after H & E-staining (right panel). Bar = 20 µm.
   (a) Co-culture with BCECF-labeled F10 cells. Some F10 cells stretched over the IVC surface (denoted by arrowheads). The fluorescent intensity of F10 cells remained as prominent as that observed after 4 h of co-culture.
   (b and c) Co-culture with BCECF-labeled BL6 cells. The fluorescent intensity of BL6 cells became much weaker than that of the 4-h co-culture; this was especially noticeable in flat-shaped BL6 cells lying over the IVC surface (denoted by arrowheads in b). An arrowhead in the right panel of c indicates a BL6 cell that is invading beneath the endothelial cell (denoted by arrows). However, it was difficult to recognize the same BL6 cell (denoted by an arrowhead in the left panel of c) with a fluorescence microscope before H & E-staining.

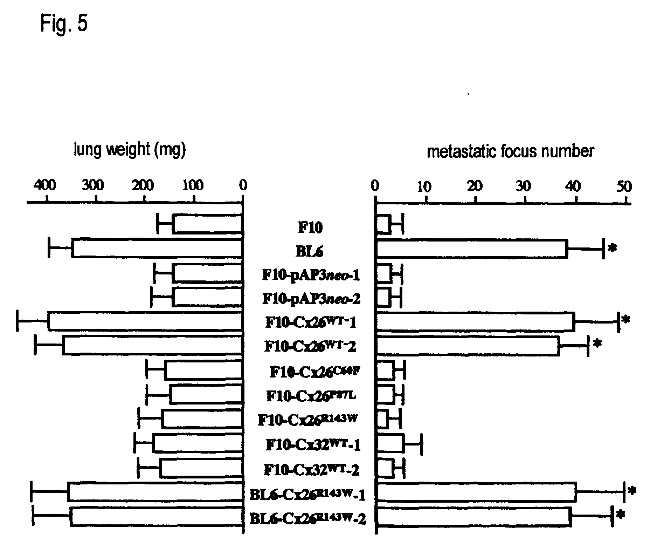

Fig. 5 shows graphs presenting the results of spontaneous metastasis assay of Cx26-transfectants. The number of colonies in the lungs produced when mice had been inoculated with F10 or BL6 cells, or with cells transfected with various forms of Cx26 and Cx32. The lung weights were also measured. Data are expressed as the mean of 7 mice per clone. Bars are standard errors. *p< 0.05 by *t*-test when compared to the values obtained from F10 cells.

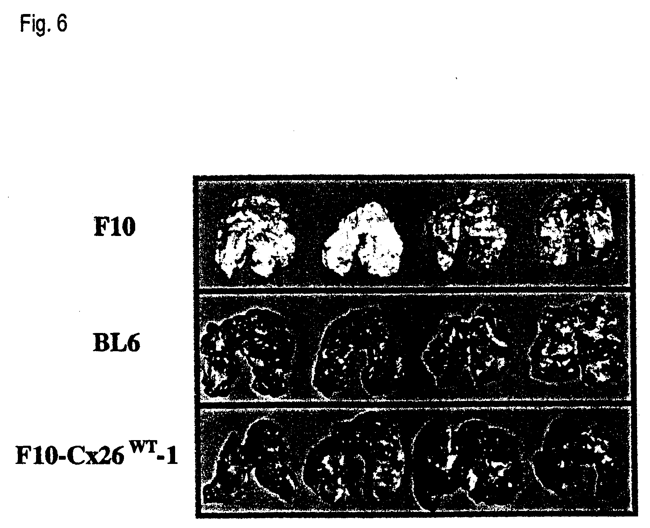

Fig. 6 shows micrographs of spontaneously metastasis locus of Cx26-transfectans. Representative results of the lung metastatic colonies produced by F10 (upper panel), BL6 (middle panel) and F10-Cx26^{WT}-1 (lower panel) cells.

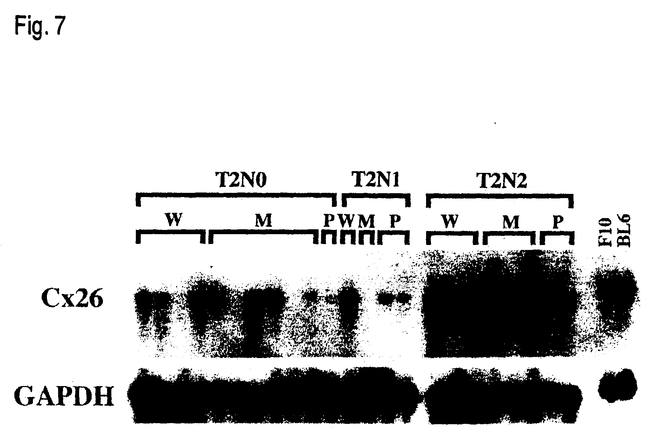

Fig 7 shows Cx26 mRNA expression in the human lung squamous cell carcinoma (SCC) detected by Nothern blot analysis. The cancer samples were divided into three groups on the basis of their N values. Total RNA from F10 and BL6 cells was also blotted. RNA loading was normalized by reprobing with a GAPDH probe. The pathologic grading of differentiation is indicated as follows: W, well; M, moderately; P, poorly.

### DETAILED DESCRIPTION OF THE INVENTION

The present invention relates to a method for testing metastatic potential comprising comparison of the expression level of connexin 26 in cancer cells with that in normal cells.

The method for testing the metastatic potential of this invention specifically refers to the following embodiments. The first embodiment is a method that quantitatively assesses what level of metastatic potential a test cancer possesses. The second embodiment is a method in which a marker for metastatic occurrence is detected. The third embodiment is a method in which a marker for metastasis prevention by targeting connexin 26 is assessed. A method for testing metastatic potential according to this invention includes all of these methods.

Human connexin 26 is a protein comprising the amino acid sequence represented by SEQ ID NO: 2. It has been elucidated that human connexin 26 is encoded by the gene having the nucleotide sequence represented by SEQ ID NO: 1 (J. Cell Biol. 118, 1213-1221, 1992). Polymorphism is often observed in nucleotide sequences of genes encoding eukaryotic proteins such as connexin 26. It is a nucleotide substitution on a small scale observed in the nucleotide sequence of a gene, and such a nucleotide substitution usually has little effect on the protein activity. Connexin 26 variants having small-scaled variations in the nucleotide and amino acid sequences due to a polymorphism and such can also be included in connexin 26 used in this invention, as long as they are involved in metastatic potential.

In this invention, connexin 26 is not necessarily limited to the one derived from humans. Besides humans, connexin homologues in mice (Eur. J. Cell Biol. 58, 81-89, 1992) or rats (J. Cell Biol. 109, (6 Pt 2), 3391-3401, 1989) are also well known. These connexin 26 homologues can be used as a marker for cancer malignancy in the corresponding animal species.

In this invention, the expression level of connexin 26 gene can be assessed based on the amount of transcribed mRNA, amount of protein or biological activity of connexin 26. These assessment procedures can be all performed according to the known methods. For example, the amount of mRNA can be quantified by RT-PCR, FISH method, or Northern blot technique. Primers and probes nesessary for these procedures can be designed based on the nucleotide sequence represented by SEQ ID NO: 1 (in the case of humans) according to the method known to those skilled in the art. Examples of primers that can be used in RT-PCR to amplify the gene encoding connexin 26, are presented below.
sense primer:
   5'-GCGAATTCAGGTGTAAATTTACCAAAAATA-3' (SEQ ID NO: 3)
antisense primer:
   5'-GCGCTCGAGCACCCAAGCTTTCCATCCTGG-3' (SEQ ID NO: 4)

When the sample is a cancer cell, mRNA can be extracted from cells to perform RT-PCR. Alternatively, the intracellular mRNA level can be directly assessed by *in situ* RT-PCR and FISH method.

Next, the amount of protein can be determined by an immunoassay using an antibody specific to connexin 26. The antibody is commercially available from, for example, Zymed, Inc. Alternatively, monoclonal and polyclonal antibodies can be obtained by the known method using a protein having the amino acid sequence represented by SEQ ID NO: 2 as an antigen. Synthetic peptides comprising amino acid sequences specific to connexin 26 selected from that represented by SEQ ID NO: 2 can also be used as an antigen. Although many variations of immunoassays are known, in the case of an immunoassay for antigenic substance, any desired method can be employed taking the processability, required sensitivity or characteristics of sample, etc. into consideration. For example, when a cancer cell is used as-it-is as a sample, the expression level of connexin 26 can be compared by conducting immunostaining for the fixed cell specimen. Alternatively, the expression level of connexin 26 can be determined by performing the ELISA method or Western blot technique using a cancer cell lysate as the sample.

The expression level of connexin 26 can be compared not only in cancer cells but also in body fluids. That is, the expression level of connexin 26 can be assessed by measuring the levels of connexin 26 mRNA and protein in the blood. In metastasis, the detachment of cancer cells into the blood stream is an important step. The amount of connexin 26 derived from cancer cells detached into the blood stream will provide important information in the metastasis test. The amount of mRNA in theblood stream can be measured by RT-PCR. The protein level can be measured by an immunoassay such as ELISA. Metastatic potential of cancer cells can be assessed by comparing the amount of mRNA (or protein) of connexin 26 thus obtained with the amount measured in healthy subjects.

Such components as described above required for assessing the expression level of connexin 26 are useful as test reagents for metastatic potential. That is, DNAs that specifically hybridize to the DNA comprising the nucleotide sequence of SEQ ID NO: 1 and comprise at least 15 nucleotides, can be used as a primer or a probe for measuring the mRNA amount of human connexin 26. Such oligonucleotides may be labeled beforehand to facilitate the detection. They are labeled by known methods with fluorescent dyes such as fluoresceine isocyanate, radioisotopes such as ³²P, or indirect labels such as digoxin. Alternatively, antibody that can specifically bind to the protein comprising the amino acid sequence of SEQ ID NO: 2 is useful for the immunological detection of the human connexin 26 protein. This antibody can also be labeled beforehand to facilitate the detection. It is labeled by known methods using enzymes such as peroxidase, fluorescent dyes such as fluoresceine isocyanate, radioisotopes such as ¹²⁵I, or indirect labels such as biotin.

Test reagents for metastatic potential according to this invention may be packaged in a kit comprising a standard sample containing connexin 26 at a predetermined concentration, a reaction buffer solution used to dilute and rinse the sample, additional reagents required for detecting labeled components, and so on.

This invention also provides, in addition to a method for testing the metastatic potential of cancer, a method for screening metastasis-suppressing agents targeting connexin 26. That is, the present invention provides a method for screening metastasis suppressors, comprising the steps of:
a) providing a protein comprising at least a domain exhibiting the gap junction activity of connexin 26;
b) contacting a candidate compound with the protein of a), and;
c) selecting a candidate compound capable of binding to a protein of a).

In this invention, connexins, besides connexin 26 derived from humans comprising the amino acid sequence of SEQ ID NO: 2, may be those derived from other animal species such as mice and rats. However, a human-derived connexin would screen compounds with a high efficacy in humans. Not only a complete molecule of connexin 26 but also only its gap-junction forming domain can be used for screening. In the human connexin 26, both the extracellular loops E1 (amino acids 41-67 numbered from the N-terminus) and E2 (amino acids 159-189 numbered from the N-terminus) are essential for the mutual connection of connexons (Cell 1996 Feb 9; 84 (3): 381-388).

In the screening method according to this invention, screening can be efficiently carried out by immobilizing either connexin 26 or a candidate compound to a solid phase and labeling the other in the mobile phase. That is, for example, a labeled candidate compound is contacted with an insoluble support material on which connexin 26 (or its gap-junction domain) is immobilized, and the binding of the two compounds can be detected based on the attachment of the label to said support material. Alternatively, compounds capable of binding to connexin 26 can be screened by contacting the labeled connexin 26 (or its gap-junction domain) with a library of candidate compounds synthesized on a solid phase by combinatorial chemistry,.

Candidate compounds that can be used in the screening method according to this invention include purified proteins (including antibodies), expression products of a gene library, a library of synthetic peptides, a RNA library, cell extracts, cell culture supernatants, or a library of synthetic low molecular weight compounds, etc., but are not limited thereto. Among them, a RNA library is preferable as candidate compounds, because a library rich in extremely wide variety can be synthesized by a relatively simple procedure. In general, synthesis of a RNA library and screening thereof can be performed as follows.
(1) First, oligonucleotides having random sequences flanked by a primer sequence (34 nucleotides) for T7RNA polymerase and a sequence of arbitrary 18 nucleotides are synthesized using a DNA synthesizer. When 25 nucleotides are flanked, a population of ologonuclotides having approximately 425≒1015 randomness can be easily synthesized.
(2) Using this population of oligonucleotides as a template and T7RNA polymerase, a population of RNA molecules having random nucleotide sequences are synthesized.
(3) RNA molecules thus synthesized are passed through a column to which the target protein has been attached under an elevated salt concentration, and then the RNA fraction adsorbed to the column is eluted under a reduced salt concentration.
(4) Complementary DNA is synthesized using RNA thus eluted as a template, said 18 nucleotide portion as a primer, and reverse transcriptase.
(5) This DNA is further amplified by PCR method.
(6) Using DNA thus amplified, processes (1)-(5) are repeated many times to purify the RNA molecule specifically binding to the target protein.

RNA molecules selected from an RNA library via these processes, which are capable of binding to connexin 26, will function as an aptamer. "Aptamer" means a functional RNA which acts by specifically binding to a target protein. Aptamer is so named for Latin "aptus" meaning "fit." In fact, aptamers targeting pigments, proteins (e.g. T4DNA polymerase), etc. have been isolated. Although the above-described procedures may be repeated using DNA itself without converting it into RNA, aptamer molecules can be obtained with much higher frequency when RNA that takes various configurations is used.

Since compounds screened by this invention, which bind to connexin 26, inhibit the gap junction through their binding to connexin 26, they can be used as active ingredients to prepare compositions for suppressing cancer metastasis.

Compounds isolated by the screening method of this invention can be formulated into pharmaceutical compositions by the known pharmaceutical manufacturing methods. These compounds may be combined with, for example, pharmacologically acceptable carriers or media (such as physiological saline, plant oil, suspending agent, surfactant, stabilizer, etc.) and administered to patients. The administration may be performed transdermally, intranasally, transtracheally, intramuscularly, intravenously or orally, depending on the properties of these compounds. Although doses may vary depending on the body weight, age and symptoms of patients as well as administration method, etc., they can be suitably selected by those skilled in the art.

By this invention, a novel marker to assess the metastatic potential is provided. Since connexin 26 is a protein involved in the cell-cell adhesion, it is assumed to participate, among the multiple steps composing metastasis, especially in the process of adhesion and invasion of cancer cells to other tissues. Therefore, by using connexin 26 as the marker, it becomes possible to quantitatively understand the metastatic potential of a cancer at its critical steps of metastasis.

In the method for screening compounds with metastasis-suppressing activity according to this invention, by targeting connexin 26, which is presumably one of the important elements supporting essential steps of metastasis, it becomes possible to search compounds capable of effectively suppressing metastasis. Compounds yielded by screening according to this invention that suppress the binding activity of connexin 26 are useful as metastasis-suppressing agents. These agents can effectively prevent metastasis of cancers expressing connexin 26 at a high level, in combination with the method for testing metastatic potential according to this invention.

This invention has demonstrated that connexin 26 is directly involved in metastasis in a subcutaneous transplantation model (spontaneous metastatic system) of melanoma, which has an extremely high metastatic potential among cancer cells. Enhancement of connexin 26 expression has been also detected in some cases of human lung small cell carcinoma. These observations indicate that connexin 26 is involved in the intravenous-invasion of highly metastatic cancer cells that call for prompt treatments, and is thought to lead to early diagnosis and anti-metastatic treatment of cancer types for which very poor treatment results have been obtained so far.

The present invention is illustrated in more detail with reference to the following examples, but is not to be construed being limited thereto.

### EXAMPLE 1

### Isolation of clones from a subtracted cDNA library

Metastasis-related genes were isolated from naturally high-metastatic mouse melanoma cell line BL6 and naturally low-metastatic cell line F10 by the subtraction method. The isolation was performed as follows.

Total RNA was extracted by the guanidine thiocyanate/CsTFA method from F10 and BL6 cells, then poly (A)⁺ RNA was purified using oligo (dT) cellulose. cDNA libraries were prepared by the linker-primer method using plasmid pAP3*neo*, and the subtracted cDNA library was constructed by subtracting the F10 cell-derived cDNA libraries from BL6 cell-derived libraries according to a known method (M. Kobori et al., Genes Cells 3:459-475, 1998). After the subtraction process was repeated four times, approximately 1500 clones were rescued. From these, the clones transcriptionally upregulated in BL6 cells compared with F10 cells were selected by Northern blot analysis and sequenced.

The DNA sequences were used to search the htgs database using the BLASTN algorithm. The result showed that a gene expressed in a high level in BL6 cells was Cx26 gene.

### EXAMPLE 2

### Colony hybridization

2 x 10⁶ colonies of the BL6 cDNA library in pAP3*neo* were transferred to nylon membrane and screened with [α -³²P]dCTP-labeled cDNA inserts containing the 3' partial sequences of Cx26, PP2A B' α, phalinin and TIB23 as probes. Plasmids were purified from the positive colonies and sequenced to confirm that the cDNA inserts encompassed the open reading frame for the four genes without mutation.

### EXAMPLE 3

### Western blot analysis

Cell lysates were prepared by alkaline treatment to enrich for integral membrane proteins according to the method described by Herzberg and Skibbents (E.L. Herzberg and R.V. Skibbents, Cell 39: 61-69, 1984). 1 µg of total cell lysate protein was separated on 10% SDS-PAGE and transferred onto Immobilon membrane (Millipore). The membrane was incubated with mouse monoclonal anti-Cx26 antibody (1:300 dilution, Zymed) for 2 h, washed with PBS, then incubated with horseradish peroxidase-conjugated anti-mouse IgG antibody (1:1000 dilution, Cappel). After washing with PBS, the blot was reacted with Renaissense chemiluminescence reagents (NEN) before exposure. A duplicate 10% SDS-PAGE gel was stained with silver according to the manufacturer's instructions (Daiichikayaku, Tokyo, Japan).

### EXAMPLE 4

### Expression of various connexins in F10 and BL6 cells

### (1) Cell lines and cell culture

Sublines of mouse melanoma B16 cells, F10 and BL6 cells were kindly provided by Dr. I.J. Fidler (The University of Texas). NIH/3T3 cells were purchased from American Type Culture Collection (ATCC). All cells were maintained in Dulbecco's modified Eagle's medium supplemented with 10% fetal calf serum (growth medium). To maintain the transfectants, the growth medium was supplemented with G-418 at a final concentration of 2 mg/ml.

### (2) Northern blot analysis.

5 µg of total RNA prepared from various clones of melanoma cells, mouse tissues or human cancer tissues were fractionated on 1% agarose-formaldehyde gels, transferred to nylon membrane, and hybridized to DNA probes labeled with [α -³²P]dCTP by the random hexamer labeling method. The Cx26 probe was prepared from a cDNA fragment encompassing the open reading frame. The probes used to detect Cx32 (Dev. Biol., 146: 117-130, 1991; nucleotides 50-582), Cx37 (J. Cell. Biol., 114: 1049-1057, 1991; nucleotides 73-940), Cx40 (J. Cell. Biol., 117: 1299-1310, 1992; nucleotides 154-826) and Cx43 (GenBank accession no. X61576; nucleotides 458-1075) were obtained by PCR. Probes for β -actin and GAPDH for standardize the mRNA amount were prepared in the same fashion. The blots were washed to a final stringency of 0.1 x SSC and 0.1% SDS at 50°C before exposure. Quantification was performed with the BAS 2000 system (Fuji).

As shown in Fig. 1a, in both F10 and BL6 cells, the Cx26 mRNA expression was easily detectable, and was upregulated 5-to 10-fold in BL6 cells. In contrast, mRNA expression levels of other connexins were below the limit of detection.

### (3) Expression of Cx26 protein in F10 and BL6 cells

A 24 kDa single Cx26 protein molecule was detected using anti-Cx26 antibody (indicated by arrow heads in Fig. 1b, right panel). Silver staining of the gel revealed that an equivalent amount of the protein was expressed (Fig. 1b, left panel). This experiment using anti-Cx26 antibody indicates that the expression of Cx26 protein in F10 and BL6 cells correlates with the expression of Cx26 mRNA.

### EXAMPLE 5

### Coculture system of inferior vena cava fragment and melanoma cells

### (1) Difference in gap junctional intercellular communication (GJIC) ability between melanoma cells and blood vessel cells

A freshly excised inferior vena cava (IVC) fragment was opened and attached to a cover glass. Then, F10 or BL6 cells were seeded onto it and co-cultured. Four hours later, the tissues co-cultured with F10 cells (Fig. 2a, left panel) or BL6 cells (Fig. 2a, right panel) were incubated with anti-Cx26 antibody and observed under a fluorescent microscope. Arrows indicate localization of Cx26 near the cell membrane. A prominent immunostaining was observed in BL6 cells but not in F10 cells. Although the majority of the immunostain was located widely in the cytoplasm of BL6 cells, some staining was observed on the BL6 cell membrane. This result suggested a formation of heterologous gap junctions between BL6 and vein cells.

### (2) Dye-transfer assay

A dye-transfer assay was performed as follows.

The membrane-permeable dye BCECF acetoxymethyl ester (Dojindo) was added to a log-phase culture at a final concentration of 1 µM. 3 h later, the growth medium was changed and the culture continued for 1 h. The cells were harvested, washed three times with PBS, and used for dye-transfer experiments. The GJIC ability was estimated as described by Mesnil et al (M. Mesnil et al., Cancer Res. 55, 629-639, 1995). To prepare the co-culture of melanoma cells and a vein fragment, the thoratic portion of the IVC was excised from freshly-killed mice. The luminal surface of the vein was exposed by making a longitudinal cut. This fragment of an opened vein was attached to a cover glass with the luminal side up by dropping liquid adhesive (Konishi) around the tissue. Then, the cover glass with the vein was placed on the bottom of a culture dish filled with growth medium containing BCECF-labeled melanoma cells (1 x 10⁶). At various timepoints, the co-culture was fixed with 4% paraformaldehyde in phosphate buffer (0.1M, pH 7.2) and observed directly by darkfield fluorescence or using a confocal laser scanning microscope. For histologic examination, unlabeled melanoma cells were used to establish the co-culture in a similar fashion.

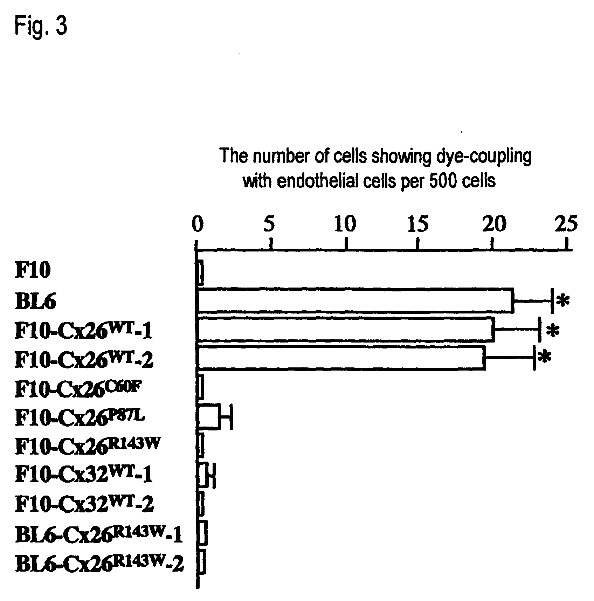

To confirm heterologous gap junction between BL6 and vein cells, the above-described dye-transfer assay was performed, in which BCECF-labeled F10 and BL6 cells were seeded onto the vein. Four hours later, the co-culture was observed under a fluorescence microscope. Transfer of BCECF is shown by arrows in Fig. 2b. The dye leaked from BL6 cells and stained the vein surface, whereas such a phenomenon was never observed in the co-culture with F10 cells. The dye-spread profile that was observed from directly above the co-culture was examined by confocal laser scanning microscope (Fig. 2c, lower panel). In the figure, arrow heads indicate BCECF-labeled F10 and BL6 cells. After 2 h, almost no dye-spread was observed from F10 or BL6 cells. At 4 h, some BL6 cells were found to have transferred the dye into surrounding cells (right-lower panel of Fig. 2c). The cells that were stained with leaked BCECF showed a cobblestone-like array, which proved an efficient dye-transfer from BL6 cells into endothelial cells of the vein. When five hundred BCECF-labeled cells were observed at 4 h, cells exhibiting dye-coupling with the endothelial cells were counted. Approximately twenty cells transferred the dye into adjacent endothelial cells (Fig. 3). On the other hand, all of the F10 cells observed retained the BCECF in their cytoplasm even at 4 h (left-lower panel of Fig. 2c).

### (3) Measurement of GJIC ability of mutant F10 cells transfected with wild-type and mutant Cx26 and Cx32

To clarify correlation between Cx26 and GJIC ability, F10 (or BL6) cells were transfected with vectors that express mutants of Cx26 mentioned below and G-418 resistant cells were selected as transformants. These mutants possess distinct dominant-negative effects on wild-type Cx26 (A. Duflot-Dancer et al., Oncogene 15, 2151-2158, 1997).
F10-Cx26^{C60F}: Cys-60 is substituted with Phe
F10-Cx26^{P87L}: Pro-87 is substituted with Leu
F10-Cx26^{R143W}: Arg-143 is substituted with Trp

GJIC ability of F10 mutants obtained by transfection with wild-type and mutant Cx26, and Cx32 was measured by the method described in (2). Two independent clones of F10 cells transfected with the wild-type Cx26 (F10-Cx26^{WT}-1 and -2) transferred the dye into endothelial cells as efficiently as BL6 cells (Fig. 3). In contrast, neither F10-Cx26^{C60F} nor F10-Cx26^{R143W} cells possessed any GJIC ability with endothelial cells, while F10-Cx26^{P87L} cells possessed only slight ability (Fig. 3).

To test for a dominant-negative effect in this assay, cDNA of Cx26^{R143W} was introduced into BL6 cells (BL6-Cx26^{R143W} -1 and -2). As expected, these two clones showed almost no GJIC ability with endothelial cells (Fig. 3). Since a heterotypic gap junction has been demonstrated between Cx26 and Cx32²³, it seemed likely that F10 clones transfected with Cx32 cDNA would be able to form gap junctions with endothelial cells. Surprisingly, neither of two such clones (F10-Cx32^{WT} -1 and -2) could do so, indicating that Cx26 may play a specific role in gap junction formation with endothelial cells (Fig. 3).

### (4) Observation of six-hour co-culture with BCECF-labeled cells

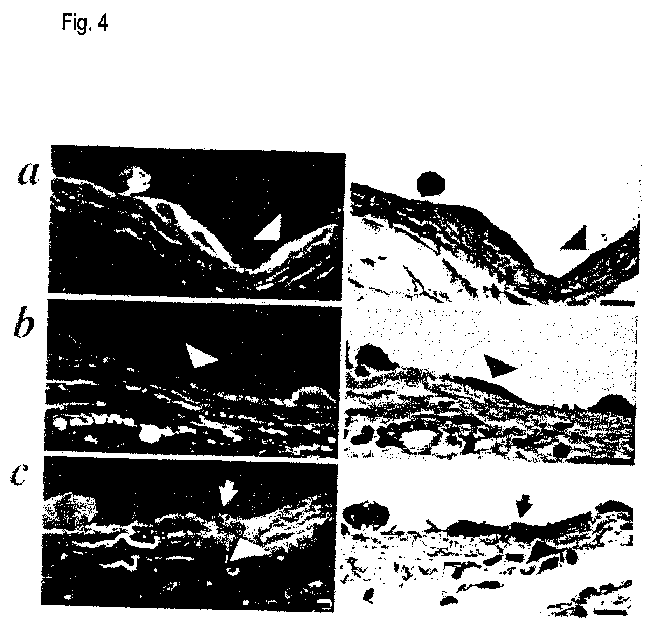

When the co-culture period was extended to 6 h, the fluorescent intensity in the cytoplasm of F10 cells remained as prominent as that observed at 4 h. This indicated a dye-coupling-deficiency of F10 cells (Fig. 4a). In contrast, the fluorescent intensity became much weaker in the majority of BL6 cells that had attached to the IVC surface, indicating an efficient dye-coupling between BL6 and the endothelial cells (Fig. 4b). Especially in the flat-shaped BL6 cells lying over the IVC surface, the intensity of fluorescence decreased dramatically. Some BL6 cells displayed an apparent invasive phenotype on the endothelial cells. Interestingly, BL6 cells invading beneath an endothelial cell had lost their fluorescence so completely that they could not be recognized by fluorescence microscope (Fig. 4c).

### EXAMPLE 6

### Spontaneous metastasis assay of transfectants

### (1) Spontaneous metastasis assay

2.5 x 10⁵ cells (F10, BL6 or their transfectant clones) were injected subcutaneously into the footpad of 4-week-old, male C57BL/6 mouse (7 mice per clone). Tumor size was monitored by direct measurement of diameter every 2 days. 3 weeks later, when tumors reached a diameter of 8-10 mm, tumor-bearing legs were amputated. The mice were allowed to survive a further 4 weeks; then autopsies were performed and the metastatic colonies formed in the bilateral lungs were counted.

### (2) Assay results

Original melanoma cells and their transfectants used in Fig. 3 were injected subcutaneously into the mouse footpad. Two months after injection, original BL6 cells were highly metastatic, while original F10 and vector-transfected F10 (F10-pAP3*neo* -1 and -2) cells were scarcely metastatic (Fig. 5). Dye-coupling-competent F10 clones, F10-Cx26^{WT}-1 and -2, were as highly metastatic as the BL6 cells. Some colonies produced by the two metastatic F10 clones looked amelanotic, contrasting with the fact that all the colonies produced by BL6 cells were solid black (Fig. 6). Dye-coupling-deficient F10 clones, F10-Cx26^{C60F}, F10-Cx26^{P87L}, F10-Cx26^{R143W}, F10-Cx32^{WT} -1 and -2, were scarcely metastatic (Fig. 5). However, dye-coupling-deficient BL6 clones, BL6-Cx26^{R143W}-1 and - 2, produced as many metastatic colonies as the original BL6 cells (Fig. 5).

### EXAMPLE 7

### Cx26 RNA Expression in human squamous cell carcinomas

### (1) Human lung cancer samples

From 1991 to 1997, 23 patients with lung squamous cell carcinoma (SCC) received a standard operation, which included the complete dissection of the first and second groups of the regional lymph nodes, in Hyogo Medical Center for Adults. The resected specimens were carefully staged according to the International Staging System for Lung Cancer³⁵. The cancer tissues were frozen before use for RNA extraction.

### (2) Cx26 RNA expression in human lung squamous cell carcinomas

To know whether Cx26 does indeed promote metastasis in human cancers, we examined Cx26 mRNA expression in SCC. 23 patients with SCC received generally similar operations including lymph node dissection. By clinicopathological staging, the M value was 0 in all the patients. Based on the T and N values, the patients were divided into three groups; low metastatic group (T2N0; n=11), moderate metastatic group (T2N1; n=4), and high metastatic group (T2N2; n=8). Total RNA extracted from the primary tumors was analysed by Northern blot (Fig. 7). In two out of eight cases belonging to the high metastatic group, a marked expression of Cx26 mRNA was detected. The expression level was found to be compatible with that of BL6 cells, after densitometric normalization using GAPDH expression. In contrast, in all the cases belonging to the low and moderate metastatic groups, Cx26 mRNA expression was below that of F10 cells.

## Claims

1. A method for testing metastatic potential, the method comprising the comparison of the expression level of the connexin 26 gene in cancer cells with that in normal cells.

2. The testing method according to claim 1, wherein the expression level of the connexin 26 gene is assessed by quantifying the amount of mRNA encoding connexin 26.

3. The testing method according to claim 2, wherein the region in said mRNA encoding at least the domain expressing the gap junction activity of connexin 26 is quantified.

4. The testing method according to claim 1, wherein the expression level of connexin 26 gene in cancer cells is measured by quantifying connexin 26 protein contained in cancer cells.

5. The testing method according to claim 4, wherein the expression level of connexin 26 gene is measured by an immunoassay using an antibody specifically recognizing the connexin 26 protein.

6. A method for testing metastatic potential, the method comprising the assessment of the expression level of the connexin 26 gene based on the amount of the humoral connexin 26 protein.

7. A reagent for testing the metastatic potential comprising a DNA that specifically hybridizes to the DNA comprising the nucleotide sequence shown in SEQ ID NO: 1, and comprising at least 15 nucleotides.

8. A reagent for testing the metastatic potential comprising an antibody that specifically binds to a protein comprising the amino acid sequence shown in SEQ ID NO: 2.

9. A method for screening metastasis suppressing agents comprising the steps of:
a) providing a protein comprising a domain that exhibits at least the gap-junction activity of connexin 26;
b) contacting said protein of a) with candidate compounds; and
c) selecting candidate compounds that bind to the protein of a).

10. The method of screening according to claim 9, wherein said candidate compounds are RNA libraries comprising random nucleotide sequences.

11. A metastasis-suppressing drug comprising a compound isolable by the method according to claim 9 or 10 as an active ingredient.
